# EUROPEAN PATENT SPECIFICATION

(11) **EP 3 236 933 B1**
(45) Date of publication and mention of the grant of the patent: **14.11.2018**
(21) Application number: 14837028.1
(22) Date of filing: 24.12.2014
(51) Int. Cl.: A61K 47/46, A61K 31/4174, A61K 9/00, A61K 31/46

(54) **A NASAL COMPOSITION CONTAINING SEA WATER AS STABILITY-IMPROVING EXCIPIENT**
NASALE ZUSAMMENSETZUNG MIT MEERWASSER ALS STABILITÄTSVERBESSERNDER HILFSSTOFF
COMPOSITION NASALE CONTENANT DE L'EAU DE MER EN TANT QU'EXCIPIENT D'AMÉLIORATION DE STABILITÉ

(43) Date of publication of application: 01.11.2017
(73) Proprietor: JADRAN - GALENSKI LABORATORIJ d.d., 51000 Rijeka (HR)
(72) Inventor: KNEZEVIC, Zdravka, HR-10000 Zagreb (HR); POPOVIC, Nina, HR-51000 Rijeka (HR); KAMBER, Silvija, HR-51000 Rijeka (HR); MAVRINAC, Marina, HR-51000 Rijeka (HR)
(74) Representative: Bihar, Zeljko
(86) International application number: PCT/HR2014/000045
(87) International publication number: WO 2016/102984

(56) References cited:
- WO-A1-99/38492
- WO-A1-2014/158119
- WO-A2-03/024433

## Description

### Technical Field

The invention is related to a nasal pharmaceutical composition containing xylometazoline hydrochloride (**1**) and ipratropium bromide (**2**) of improved stability due to use of purified sea water as stability-improving excipient.

### Technical Problem

Technical problem is related to stability of xylometazoline hydrochloride (**1**) and ipratropium bromide (**2**) in pharmaceutical compositions for nasal use.

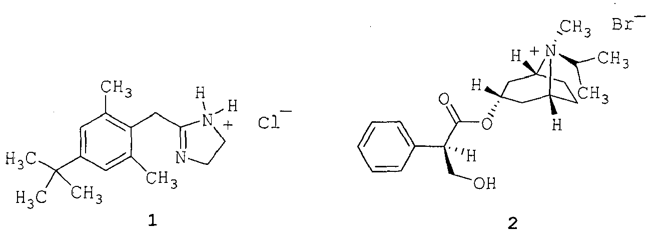

Nasal compositions are usually based on very dilute aqueous solutions, often with various excipients with role of co-solvent, tonicity adjusting agent, buffering agent, preservative, antioxidant, stabilizer, suspending agent, chelating agent, pH adjusting agent, penetration enhancer, surfactant, and humectant. Such medium, more or less causes significant acceleration of chemical degradation of active pharmaceutical substances such as (**1**) and especially (**2**), which are prone to hydrolytic degradation. Such process can often lead to out-of-specification results in pharmaceutical industry, which requires necessary re-formulation work in order to improve chemical stability of the compositions through the shelf life, usually 2 years at room conditions, or more.

The technical problem that is solved by the present invention can be regarded as significant improvement in chemical stability of topical nasal compositions containing xylometazoline hydrochloride (**1**) and ipratropium bromide (**2**).

### Previous State of Art

Compound 2-[4-(1,1-Dimethylethyl)-2,6-dimethylbenzyl]-4,5-dihydro-1*H*-imidazole hydrochloride, known under generic name of xylometazoline hydrochloride (**1**), is a well-known and widely used pharmaceutical active substance (API) of α-adrenergic activity. Thus, it acts as vasoconstrictor, with most important practical use as nasal decongestant.

Compound [8-Methyl-8-(1-methylethyl)-8-azoniabicyclo[3.2.1]oct-3-yl]-3-hydroxy-2-phenylpropanoate], known under generic name ipratropium bromide (**2**), is also a well-known API of parasympatholytic, anticholinergic activity that is employed as bronchodilator and antiarrhythmic.

Xylometazoline hydrochloride (**1**) is API that is mainly employed for preparation of nasal drugs. For instance, document WO 00/78297 A2; applicant Boehringer Ingelheim, Germany; discloses chemically and microbiologically stable 0.01-1% xylometazoline solution for nasal use, which is based on stabilization of xylometazoline hydrochloride (**1**) by humectants glycerol (2.0-2.8%) or sorbitol (3.5-4.5%) and inorganic (phosphate) or organic buffer (trometamol), with overall content of excipients from 1-10%, and pH of 4.5-7.5.

Similarly, document WO 2005/018601 A1; applicant Merck Patent GmbH, Germany; teaches that aqueous solution of xylometazoline hydrochloride ((**1**); 0.005-1%) can be stabilized with buffer salts (0.01-3%) in the presence of zinc salts (0.1-10%) suitable for nasal use, with final pH value of the formulation of 5.0-7.2.

Also, document EP 0773022 B3; applicant M.C.M. Klosterfrau Vertrieb GmbH, Germany; discloses stable xylometazoline hydrochloride ((**1**); 0.01-0.1%) solution for nasal use which is based on its formulation with panthenol (0.2-10%). The pH value of such formulation is not specified, but the value of pH 5.3 is mentioned in the patent specification.

Beside its vitamin B5 action, panthenol is also a humectant, which may also stabilize xylometazoline hydrochloride (**1**) against hydrolysis in dilute aqueous solution by forming several possible hydrogen bonds.

Additionally, document DE 10000612 A1; inventor M. Becker; teaches about the preservative action of 1% sodium chloride (NaCl) on dilute aqueous solution of xylometazoline hydrochloride ((**1**); 0.01-0.1%), which serves as pharmaceutical composition for nasal use. This document is focussed on microbiological stabilization of dilute aqueous solution of xylometazoline hydrochloride, whilst no possible effect of NaCl against chemical stability was studied or mentioned.

The topical nasal use of ipratropium bromide (**2**) is also known in the art, e.g. from scientific documents:
(1) P. Borum, L. Olsen, B. Winther, N. Mygind: Ipratropium Nasal Spray: A New Treatment of Rhinorrhea in the Common Cold, Am. Rev. Respir. Dis. 123 (1981) 418-420; and
(2) P. Borum: Nasal disorders and anticholinergic therapy, Postgrad. Med. J. 63 (1987) 61-68.

These documents also describe parallel application of alpha-sympathomimetic drugs such as xylometazoline hydrochloride (**1**) with ipratropium bromide (**2**) for the treatment of the common cold and rhinorrhea.

The use of the purified sea water with xylometazoline hydrochloride (1) is known in the art. For instance, document DE10027474 A1; applicant Stada Arzneimittel AG, Germany; discloses a pharmaceutical composition containing: (a) sea water, (b) alpha-sympathomimetic agent such as xylometazoline hydrochloride, and, optionally, (c) water; for treatment of swelling and inflammation of nasal mucosa due to the common cold or hay fever.

However, in this document, nothing about the influence of sea water on chemical stability of xylometazoline hydrochloride (**1**) is studied or even mentioned.

Document EP1091747 B1; applicant Goemar Lab SA, France; discloses the use of sea water aerosol for treatment of inflammatory processes of bronchial or pulmonary mucosa. The sea water was of the following characteristics: iso-osmotic; dry matter 1-2% w/w; osmolality 305-315 mOsm/kg; pH= 7.8-8.3; density of 1.008-1.01 g/ml; and the composition: sodium 2.000-2.600 mg/l, potassium 40-80 mg/l, chloride 5.800-6.000 mg/l, calcium 300-400 mg/l, and magnesium 1.200-1.500 mg/l.

Sea water has a well-established history of use in nasal medicinal products weather they are registered and marketed in category of medical devices, e.g. Sterimar line, Aqua Maris line etc., or as pharmaceutical drugs, e.g. Snup® (STADA GmbH), Mar Rhino® (Stada Arzneimittel AG), Xyladur (Premier Research GmbH), Meralys® (Jadran Galenski Laboratorij).

According to our best knowledge, the closest prior art document is WO 03/024422; applicant Nycomed A/S, Denmark. It discloses the composition for treatment of common cold comprising xylometazoline hydrochloride (**1**) and ipratropium bromide (**2**), or, alternatively other xylometazoline and ipratropium salts, in the form of aqueous solution for topical nasal use of pH 3-7. This initial pH range of the composition was reduced in European patent equivalent EP 1446119 B1 to the pH range of 4.2-5.8 during the examination process.
Among special pharmaceutical excipients, the composition cited in document WO 03/024422 uses glycerol as humectant and disodium ethylene diaminotetraacetate (edetate) dihydrate (Na₂EDTA•2H₂O) as a complex binder (chelating agent).

The composition disclosed in WO 03/024422 is used for treatment of symptoms associated with the common cold, rhinitis, as well as nasal congestion, sneezing, and hypersecretion (rhinorrhea).

The composition for topical nasal use based on dilute aqueous solution of xylometazoline hydrochloride (**1**) and ipratropium bromide (**2**) with improved chemical stability, thanks to the stability- improving action of purified sea water, seems to represents a novel and inventive solution in comparison with the prior art documents.

### Summary of Invention

The present invention discloses the pharmaceutical composition for topical nasal use containing xylometazoline hydrochloride (**1**), 0.01-0.1% w/w; ipratropium bromide (**2**), 0.01-0.1% w/w; as active pharmaceutical ingredients (APIs) as well as excipients required to yield final dosage forms of nasal drops, liquid nasal spray, or nasal wash, in which.

The stability of APIs are increased by the use of purified sea water as functional, stability-improving excipient, in amounts of 5-25% w/w. A nasal composition has osmolality within the range 270-820 mOsm/kg, the pH value within the range 3-7, preferably within the range 3-4.2 or 5.8-7.0, and most preferably within the range 3.2-4.2 where it shows improved stability.

Prepared solution is filled into various multiple or unit dose container closure systems, as known in the art to be used for nasal application. The latter can be glass or plastic containers equipped by droppers, dropper pumps or spray pumps, nasal injection or aerosols equipped by different dosing systems. Container closure systems in specific arrangement, e.g. APF Plus®, 3K® system, airless and non-airless, generally known as preservative free systems which support preservative-free formulations.

### Brief Description of the Drawings

Figure 1 - shows flowcharted preparation of composition according to the present invention.
Figure 2 - shows probable structures of complexes of xylometazoline hydrochloride (**1**) and ipratropium bromide (**2**) with magnesium (Mg²⁺) and calcium (Ca²⁺) salts, whose generation within the composition from the invention presumably contribute to their improved resistance against hydrolytic degradation.

### Detailed Description

The invention discloses an improved pharmaceutical composition of xylometazoline hydrochloride (**1**) and ipratropium bromide (**2**) in pharmaceutical compositions for nasal use. 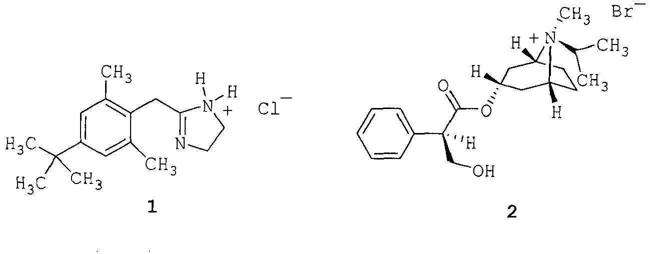 We have found that purified sea water does stabilize the composition against chemical degradation of APIs. Beside the fact that certain ingredients of sea water, namely physiologically important cations and anions, do act positively on the health of nasal mucosa, some of them evidently stabilize the active pharmaceutical ingredients (APIs) **1** and **2** of the composition.

The composition from the present invention is consisting of:
(i) xylometazoline hydrochloride (**1**), 0.01-0.1% w/w;
(ii) ipratropium bromide (**2**), 0.01-0.1% w/w;
(iii) pharmaceutical excipients required to form final dosage forms of nasal drops, liquid nasal spray, or nasal wash; and
(iv) purified sea water as functional, stability-increasing excipient, 5-25% w/w; which is characterized by pH value of 3-7, and said nasal composition has osmolality within the range 270-820 mOsm/kg.

Alternatively, the preferred pH value of the composition from the invention is characterized by pH value of 3-4.2 or 5.8-7.0.

Furthermore, the most preferred pH value of the composition from the invention is characterized by pH value of 3.2-4.2.

Typical osmolalities measured for the formulation of the present invention, depending on the possible range of key ingredients composition, are between 270-820 mOsm/kg.

More specifically, the preferred composition, according to the present invention, is consisting of:
(i) xylometazoline hydrochloride (**1**), 0.05% w/w;
(ii) ipratropium bromide (**2**), 0.06% w/w;
(iii) purified sea water as functional, stability-improving excipient, 10% w/w; and
(iv) pharmaceutical excipients required to form final dosage form of nasal drops or liquid nasal spray; with the pH value within the range 3.2-4.2.

Xylometazoline hydrochloride (**1**) in the present invention is used in all available crystalline forms.

Ipratropium bromide (**2**) that is employed in the present invention is ipratropium bromide, anhydrous or monohydrate, in all available crystalline forms.

Purified sea water that is used in the composition of the invention is manufactured from deep Adriatic Sea water by several filtration steps in order to remove both various marine organisms and microorganisms. Since the process for manufacturing of purified sea water is not so commonly known, detailed procedure of its preparation is given in Example 1.

Purified sea water is quantitatively analysed by ion chromatography as known in the art, e.g. from scientific documents:
(3) T. Bolanca, S. Cerjan-Stefanovic, M. Regelja, D. Stanfel: Ion Chromatographic Method Development for Monitoring Seawater Quality Used in Over-The-Counter Pharmaceutical Industry, J. Sep. Sci. 28 (2005) 1476-1484; and
(4) T. Bolanca, S. Cerjan-Stefanovic, M. Regelja, D. Stanfel: Development of an Ion Chromatographic Method for Determination of Inorganic Cations in Seawater used in OTC Pharmaceutical Industry, J. Liq. Chrom. 28 (2005) 249-260.

Method of analysis of the purified sea water is described in Example 1. Sea water naturally contains sodium (Na⁺), magnesium (Mg²⁺), potassium (K⁺), calcium (Ca²⁺), chloride (Cl⁻), sulphate (SO₄²⁻), hydrogencarbonate (HCO₃⁻), bromide (Br⁻), and other physiologically important cations and anions in trace amounts.

Typical specification of the content of cations and anions in purified sea water is given in Table 1.

**Table 1. Specification of the compositions of cations and anions in purified sea water used in the composition of the present invention.**

| **No.** | **Ingredient** | **Requirement (mg/ml)** |
|---|---|---|
| *Cations:* | | |
| 1 | Sodium (Na⁺) | NLT 7.50 |
| 2 | Magnesium (Mg²⁺) | NLT 1.00 |
| 3 | Calcium (Ca²⁺) | NLT 0.25 |
| 4 | Potassium (K⁺) | NLT 0.20 |

| *Anions:* | | |
|---|---|---|
| 5 | Chlorides (Cl⁻) | NLT 16.50 |
| 6 | Sulfates (SO₄²⁻) | NLT 1.80 |
| 7 | Hydrogencarbonates (HCO₃⁻) | NLT 0.10 |
| 8 | Bromides (Br⁻) | NLT 0.04 |
| NLT = not less than | | |

Beside additional pharmaceutical purified water, purified sea water within the composition of the present invention serves as an excipient, a co-diluent and a tonicity adjusting agent. Moreover, purified sea water shows stability-improving effects in both API **1** and **2** of the composition. Thus, purified sea water is further termed as *stability-improving excipient,* as proved and described in further disclosure of this invention.

In this manner, the term "*improving*" means significantly higher chemical stability of active pharmaceutical ingredients (APIs) xylometazoline hydrochloride (**1**) and ipratropium bromide (**2**), within the formulation of the present invention, against hydrolytic degradation. This "*improvement*" was achieved by using purified sea water, which showed this stability-improving effect, as is described in the following part of this application.

Other pharmaceutical excipients that are used in the composition from the present invention are selected from the group consisting of:
(i) humectants;
(ii) chelating agents;
(iii) preservatives; this are used optionally, depending on container-closure system used for filling;
(iv) pH-adjusting agents; and
(v) purified water.

Humectants are selected from the group consisting of: glycerol, 1,2-propylene glycol, sorbitol, d-panthenol, ectoin, other commonly known pharmaceutically acceptable humectants, or their mixtures. Humectants are used in concentrations of 2-5% w/w of the composition.

Chelating agents are selected from the group consisting of: sodium, or potassium salts of ethylenediaminotetraacetic (edetic) acid (EDTA), diethylenetriamine pentaacetic acid (DTPA), nitrilotriacetic acid (NTA), other common pharmaceutically acceptable chelating agents, or their mixtures. Representative example of such chelating agents is disodium edetate dihydrate (Na₂EDTA•2H₂O). Chelating agents are typically employed in concentrations of 0.001-0.1% w/w of the composition.

Preservatives are selected from the group consisting of: quaternary ammonium salts such as benzalkonium chloride; parabens like methyl 4-hydroxybenzoate, ethyl 4-hydroxybenzoate, propyl 4-hydroxy benzoate, or their mixtures; benzoic acid; sorbic acid; benzyl alcohol; 2-phenylethyl alcohol; other common pharmaceutically acceptable preservatives; or their mixtures.

Finally, pH-adjusting agents are selected from the group consisting of:
(i) acids: hydrochlorid acid (HCl) and sulphuric acid (H₂SO₄); and
(ii) bases: sodium hydroxide (NaOH), sodium carbonate (Na₂CO₃), and sodium hydrogencarbonate (NaHCO₃), potassium hydroxide (KOH), potassium carbonate (K₂CO₃), and potassium hydrogencarbonate (KHCO₃); and
(iii) other inorganic and organic pharmaceutically acceptable inorganic and organic acids and bases.

In the case of the use of hydrochlorid acid (HCl), diluted solution of HCl in purified water of concentrations of 0.1-6 mol/dm³ is employed. In the case of the use of sulphuric acid (H₂SO₄), diluted solution in purified water of concentration of 0.1-3 mol/dm³ is applied.

pH-adjusting agents are used in *quantum satis* (q.s.) principle to adjust the pH value up to the required level from 3 to 7.

Purified water that is used in the composition of the present invention meets the requirements of European pharmacopoeia 8.0, p.3561-3563 for pharmaceutical water that can be used also in nasal products.

### Preparation of the composition from the present invention

The composition of the present invention is prepared through the following manufacturing steps:
(i) filtration of purified sea water through 0.2 µm filter; for this purpose resin-bonded glass fiber filter is used;
(ii) mixing of filtered, purified sea water with predominant part of purified water;
(iii) dissolution of excipients; humectants like glycerol and chelating agent such as Na₂EDTA•2H₂O;
(iv) dissolution of active pharmaceuticals ingredients (APIs): xylometazoline hydrochloride (**1**) and ipratropium bromide (**2**);
(v) filtration through 1.2 µm filter; for this purpose polypropylene filter is employed; this is performed for removal of eventual traces of mechanical impurities and insolubles that originates from APIs and excipients;
(vi) determination of pH value of thus prepared solution;
(vii) correction of pH to a defined value by addition of pH-adjusting agent; e.g. dilute solution of HCl or NaOH;
(viii) addition of remaining part of purified water up to the final mass of the batch;
(ix) filtration through 0.2 µm filter for bioburden reduction; for this filtration polyvinylidene difluoride (PVDF) filter is used;
(x) sterile filtration through 0.1 µm filter; for this filtration polyvinylidene difluoride (PVDF) filter is employed; and
(xi) filling the filtered liquid composition into containers.

All filtration processes (i), (v), (ix) and (x) are performed by the means of pressure filtration using maximal pressure of 5.3 bar of sterile air. For critical filtrations in steps (ix), reduction of bioburden, and (x), sterile filtration, regular testing of filters integrity are carried out in order to ensure proper quality of filtration processes and sterility of the final product.

Prepared solution is filled into various multiple or unit dose container closure systems, as known in the art to be used for nasal application. The latter can be glass or plastic containers equipped by droppers, dropper pumps or spray pumps, nasal injection or aerosols equipped by different dosing systems. Container closure systems in specific arrangement, e.g. APF Plus®, 3K® system, airless and non-airless, generally known as preservative free systems which support preservative-free formulations.

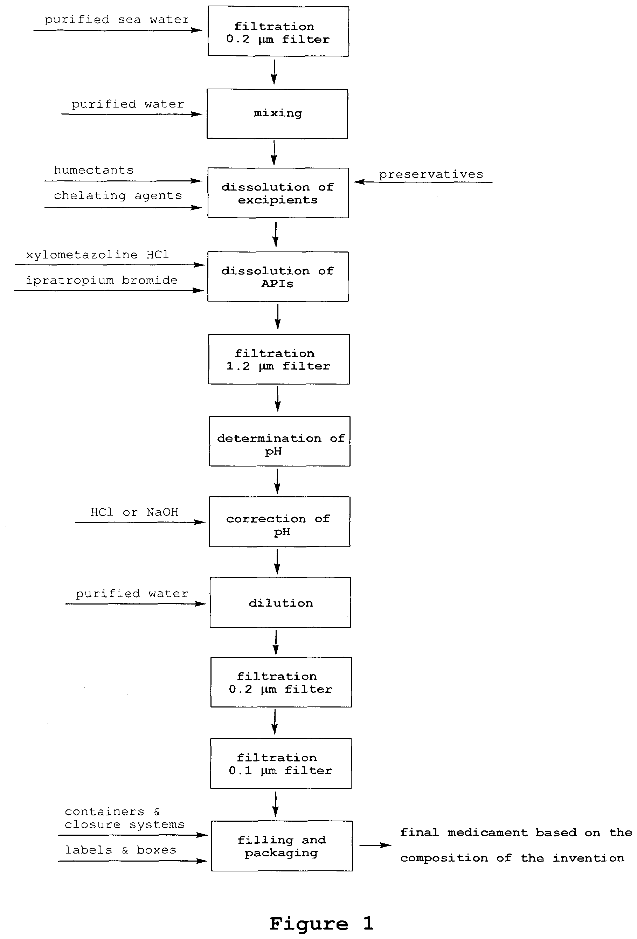

The process for production of the composition from the present invention is schematically shown in Figure 1.

Preparations of representative examples of the composition from the present invention are disclosed in experimental Examples 3-11.

### Stability results of the composition

The stability of the composition according to the invention was primarily studied by monitoring of degradation products that appeared under turbo-accelerated conditions, at 50 °C and 80% of relative humidity (RH) at starting point (t₀), after 14, and 28 days; as is usual in the pharmaceutical industry.

Turbo-accelerating conditions are more challenging in terms of increased temperature (50°C) and relative humidity (80% RH) in order to accelerate stability behaviour in shorter period of time, e.g. 28 days.

Additionally, stability testings were performed also under standard ICH* stability conditions, such as:
(a) accelerated studies at 40°C and 75% RH;
(b) intermediate studies at 30 °C and 65% RH; and
(c) standard, room temperature conditions studies at 25 °C and 65% RH.
*The International Conference on Harmonisation of Technical Requirements for Registration of Pharmaceuticals for Human Use (**ICH**)

Detailed procedure of stability studies of the composition from the present invention is described in Example 12.

According to the monograph of xylometazoline hydrochloride (**1**) from European pharmacopoeia (Ph.Eur. 8.0), among other related substances, the most important, first degradation product formed by hydrolysis is *N*-(2-aminoethyl-2-[4-(1,1-dimethylethyl)-2,6-dimethyl phenyl]acetamide (**1A**), therein designated as impurity A:

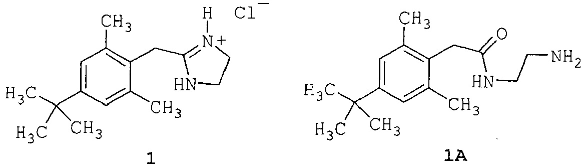

Among the related substances of ipratropium bromide (**2**) as described in the corresponding monograph of European pharmacopoeia (Ph.Eur. 8.0), the most important impurities that come from degradation of **2** are:
(1) (1*R*,3r,5*S*,8*r*)-3-hydroxy-8-methyl-8-(1-methylethyl)-8-azonia bicyclo[3.2.1]octane (**2A**);
(2) (1*R*,3*r*,5*S*,8*s*)-3-[[(2*RS*)-3-hydroxy-2-phenylpropanoyl]oxy]-8-methyl-8-(1-methylethyl)-8-azoniabicyclo[3.2.1]octane (**2B**);
(3) (2*RS*)-3-hydroxy-2-phenylpropanoic acid (**2C**), known under generic name DL-tropic acid; and
(4) 2-phenylpropenoic acid (**2D**), known under generic name atropic acid:

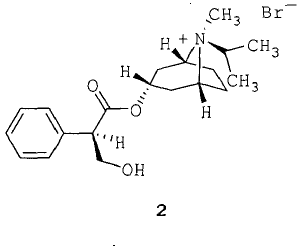

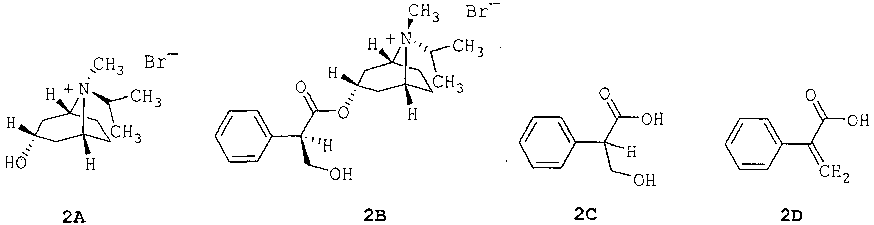

As control formulation that represents the state-of-the-art, the composition without the purified sea water was employed; product of Example 2. Compositions of studied formulation from the invention, i.e. product of Example 3, and the control formulation, product of Example 2, are given in Table 2.

**Table 2. Compositions of model formulation of the present invention and the control formulation.**

| **No.** | **Ingredient** | **State-of-the-art (Example 2)** | **The invention (Example 3)** |
|---|---|---|---|
| 1 | xylometazoline hydrochloride (**1**) | 0.05 | 0.05 |
| 2 | ipratropium bromide (**2**) | 0.06 | 0.06 |
| 3 | glycerol, 85% | 2.73 | 2.73 |
| 4 | Na₂EDTA•2H₂O | 0.05 | 0.05 |
| 5 | purified sea water | - | 10.00 |
| 6 | 0.1 M HCl/NaOH | q.s.¹ | q.s.¹ |
| 7 | purified water | ad 100.00 g | ad 100.00 g |

| | | | |
|---|---|---|---|
| ¹ q.s. = *quantum satis* to pH= 3-7; specific examples includes several specific values of pH 6.0 and pH 4.0. | | | |

During the stability study, in both compositions the content of active pharmaceutical ingredients (APIs) **1** and **2** were monitored, as well as impurity **1A** of API **1,** impurities **2A-2D** of API **2,** other impurities, and total impurities of the compositions.

All analyses were performed by ultra high performance liquid chromatography (UHPLC) as described in Example 12.

The results of the assay of active pharmaceutical ingredients xylometazoline hydrochloride (API **1**) and ipratropium bromide (API **2**) of the compositions during stability testings are presented in Table 3.

**Table 3. Stability results of the composition of the present invention (Example 3) containing 10% w/w of purified sea water in comparison with the composition without purified sea water (Example 2) at pH= 6.0; the assay of APIs in the compositions after being stored at turbo-accelerated conditions.**

| **No.** | **Stage** | **pH (5-6)** | **Assay of API 1 (%)¹** | **Conc. of API 1 (mg/ml)²** | **Assay of API 2 (%)³** | **Conc. of API 2 (mg/ml)⁴** |
|---|---|---|---|---|---|---|
| **The control composition (Example 2)** | | | | | | |
| 1 | t₀ | 6.00 | 98.8 | 0.494 | 98.5 | 0.591 |
| 2 | 14 days | 5.81 | 99.3 | 0.497 | 95.1 | 0.571 |
| 3 | 28 days | 5.67 | 96.0 | 0.480 | 90.3 | 0.542 |

| **The composition of the present invention (Example 3)** | | | | | | |
|---|---|---|---|---|---|---|
| 1 | t₀ | 5.97 | 100.9 | 0.505 | 100.4 | 0.602 |
| 2 | 14 days | 5.43 | 100.1 | 0.501 | 98.2 | 0.589 |
| 3 | 28 days | 5.35 | 98.5 | 0.492 | 96.7 | 0.580 |

| | | | | | | |
|---|---|---|---|---|---|---|
| ¹ Specification involves limits of assay of xylometazoline hydrochloride (**1**) of 95.0-105.0% w/w. ² Specification includes allowed variations of concentration (conc.) of xylometazoline hydrochloride (**1**) in the composition within the limits of 0.475-0.525 mg/ml. ³ Specification involves limits of assay of ipratropium bromide (**2**) of 95.0-105.0% w/w. ⁴ Specification includes allowed variations of concentration (conc.) of ipratropium bromide (**2**) in the composition within the limits of 0.57-0.63 mg/ml. | | | | | | |

Furthermore, stability profile of related substances (impurities) that were formed upon solutions being stored at turbo-accelerated conditions in the compositions is given in Table 4.

**Table 4. Stability results of the composition of the present invention (Example 3) containing 10% w/w of purified sea water in comparison with the composition without purified sea water (Example 2) at pH= 6.0; the content of impurities in the compositions.**

| **No.** | **Stage** | **Impurity** | | | | | | |
|---|---|---|---|---|---|---|---|---|
| | | **1A (%)¹** | **2A (%)²** | **2B (%)³** | **2C (%)³** | **2D (%)³** | **UI (%)⁴** | **TI (%)⁵** |
| **The control composition (Example 2)** | | | | | | | | |
| 1 | t₀ | <0.10 | 0.003 | <0.10 | <0.10 | <0.10 | <0.10 | <0.10 |
| 2 | 14 days | 0.16 | - | <0.10 | 1.87 | <0.10 | <0.10 | 2.03 |
| 3 | 28 days | 0.31 | 0.34 | <0.10 | 3.39 | <0.10 | 0.18 | 3.88 |

| **The composition of the present invention (Example 3)** | | | | | | | | |
|---|---|---|---|---|---|---|---|---|
| 1 | t₀ | <0.10 | 0.003 | <0.10 | 0.19 | <0.10 | <0.10 | 0.19 |
| 2 | 14 days | <0.10 | - | <0.10 | 0.97 | <0.10 | <0.10 | 0.97 |
| 3 | 28 days | 0.13 | 0.11 | <0.10 | 1.50 | <0.10 | 0.13 | 1.76 |

| | | | | | | | | |
|---|---|---|---|---|---|---|---|---|
| ¹ Specification involves limit of not more than 0.3% of impurity A of xylometazoline hydrochloride (**1**). ² Specification involves limit of not more than 0.2% of impurity A of ipratropium bromide (**2**). ³ The limit for impurities **2B, 2C,** and **2D** is not more than 0.2%. ⁴ UI = unspecified impurities; limit is not more than 0.2%. ⁵ TI = total impurities; limit is not more than 1.0%. | | | | | | | | |

These results clearly demonstrate that the composition from the present invention (Example 3) is significantly more stable, obviously thanks to the content of purified sea water (10% w/w), what is completely unexpected to the person skilled in the art.

One can be aware that the specification limits are only informative for turbo-accelerated storage condition stability data, while they are very relevant for ICH storage condition stability data.

Increased stability is obvious from the results of the assay of APIs **1** and **2** wherein significant improvements in stability of both xylometazoline hydrochloride (**1**) from 96.0% to 98.5% as well as ipratropium bromide (**2**) from 90.3% to 96.7% were obtained (please inspect data in Table 3).

These improvements are consequences of remarkable stability-improving action of purified sea water, what can be seen from the results of quantitative UHPLC determination of impurities, wherein the most significant differences were observed in decreased amounts of impurities **1A** (0.31% to 0.13%), **2A** (0.34% to 0.11%), **2C** (3.39% to 1.50%), various unspecified impurities (UI; 0.18% to 0.13%), and total impurities (TI; 3.88 to 1.76) (Table 4).

The stability of the composition from the present invention was further proved by additional stability studies that confirmed substantial stability of the composition over wide range of pH from 3-7. The results are presented in Tables 5 and 6.

**Table 5. The stability results of the composition of the present invention (Example 4) over a range of pH values; the assay of APIs in the composition, after being stored at turbo-accelerated conditions.**

| **No.** | **Stage** | **pH** | **Assay of API 1 (%)¹** | **Conc. of API 1 (mg/ml)²** | **Assay of API 2 (%)³** | **Conc. of API 2 (mg/ml)⁴** |
|---|---|---|---|---|---|---|
| **The composition of the present invention of pH= 3** | | | | | | |
| 1 | t₀ | 3.02 | 99.4 | 0.497 | 98.9 | 0.594 |
| 2 | 14 days | 3.05 | 100.7 | 0.503 | 99.9 | 0.600 |
| 3 | 28 days | 3.01 | 98.3 | 0.492 | 99.2 | 0.595 |

| **The composition of the present invention of pH= 4** | | | | | | |
|---|---|---|---|---|---|---|
| 1 | t₀ | 4.08 | 100.7 | 0.504 | 100.2 | 0.601 |
| 2 | 14 days | 4.10 | 101.5 | 0.508 | 100.9 | 0.605 |
| 3 | 28 days | 4.03 | 100.1 | 0.501 | 101.5 | 0.609 |

| **The composition of the present invention of pH= 5** | | | | | | |
|---|---|---|---|---|---|---|
| 1 | t₀ | 5.14 | 99.7 | 0.498 | 99.3 | 0.596 |
| 2 | 14 days | 4.49 | 101.3 | 0.506 | 100.8 | 0.605 |
| 3 | 28 days | 4.30 | 98.9 | 0.494 | 99.4 | 0.596 |

| **The composition of the present invention of pH= 7** | | | | | | |
|---|---|---|---|---|---|---|
| 1 | t₀ | 6.65 | 99.0 | 0.495 | 98.2 | 0.589 |
| 2 | 14 days | 4.60 | 100.3 | 0.502 | 98.3 | 0.590 |
| 3 | 28 days | 4.44 | 98.7 | 0.493 | 98.4 | 0.591 |

| | | | | | | |
|---|---|---|---|---|---|---|
| ¹ Specification involves limits of assay of xylometazoline hydrochloride (**1**) of 95.0-105.0% w/w. ² Specification includes allowed variations of concentration (conc.) of xylometazoline hydrochloride (**1**) in the composition within the limits of 0.475-0.525 mg/ml. ³ Specification involves limits of assay of ipratropium bromide (**2**) of 95.0-105.0% w/w. ⁴ Specification includes allowed variations of concentration (conc.) of ipratropium bromide (**2**) in the composition within the limits of 0.57-0.63 mg/ml. | | | | | | |

**Table 6. The stability results of the composition of the present invention (Example 4); the content of impurities in the compositions, after being stored at turbo-accelerated conditions.**

| **No.** | **Stage** | **pH** | **Impurity** | | | | | | |
|---|---|---|---|---|---|---|---|---|---|
| | | | **1A (%)¹** | **2A (%)²** | **2B (%)³** | **2C (%)³** | **2D (%)³** | **UI (%)⁴** | **TI (%)⁵** |
| **The composition of the present invention of pH= 3** | | | | | | | | | |
| 1 | t₀ | 3.02 | <0.10 | n.a. | <0.10 | <0.10 | <0.10 | <0.10 | <0.10 |
| 2 | 14 days | 3.05 | <0.10 | n.a. | <0.10 | <0.10 | <0.10 | <0.10 | <0.10 |
| 3 | 28 days | 3.01 | <0.10 | n.a. | <0.10 | <0.10 | <0.10 | <0.10 | <0.10 |

| **The composition of the present invention of pH= 4** | | | | | | | | | |
|---|---|---|---|---|---|---|---|---|---|
| 1 | t₀ | 4.08 | <0.10 | n.a. | <0.10 | <0.10 | <0.10 | <0.10 | <0.10 |
| 2 | 14 days | 4.10 | <0.10 | n.a. | <0.10 | <0.10 | <0.10 | <0.10 | <0.10 |
| 3 | 28 days | 4.03 | <0.10 | n.a. | <0.10 | <0.10 | <0.10 | <0.10 | <0.10 |

| **The composition of the present invention of pH= 5** | | | | | | | | | |
|---|---|---|---|---|---|---|---|---|---|
| 1 | t₀ | 5.14 | <0.10 | n.a. | <0.10 | <0.10 | <0.10 | <0.10 | <0.10 |
| 2 | 14 days | 4.49 | <0.10 | n.a. | <0.10 | 0.12 | <0.10 | <0.10 | 0.12 |
| 3 | 28 days | 4.30 | <0.10 | n.a. | <0.10 | 0.22 | <0.10 | <0.10 | 0.22 |

| **The composition of the present invention of pH= 7** | | | | | | | | | |
|---|---|---|---|---|---|---|---|---|---|
| 1 | t₀ | 6.65 | <0.10 | n.a. | <0.10 | 0.11 | <0.10 | <0.10 | 0.11 |
| 2 | 14 days | 4.60 | <0.10 | n. a. | <0.10 | 0.51 | <0.10 | <0.10 | 0.51 |
| 3 | 28 days | 4.44 | <0.10 | n. a. | <0.10 | 0.63 | <0.10 | <0.10 | 0.63 |

| | | | | | | | | | |
|---|---|---|---|---|---|---|---|---|---|
| n.a. = not analysed. ¹ Specification involves limit of not more than 0.3% of impurity A of xylometazoline hydrochloride (**1**). ² Specification involves limit of not more than 0.2% of impurity A of ipratropium bromide (**2**)**.** ³ The limit for impurities **2B, 2C,** and **2D** is not more than 0.2%. ⁴ UI = unspecified impurities; limit is not more than 0.2%. ⁵ TI = total impurities; limit is not more than 1.0%. | | | | | | | | | |

These results strongly support the conclusion that the composition from the present invention is stable under a wide range of pH values from 3 to 7, what is seen both from the assay of APIs **1** and **2** (Table 5), as well as from the quantitative HPLC determination of impurities formed from degradation of APIs **1** and **2** (Table 6).

Although these stability testing were not analysed against the content of impurity **2A,** stability improving effect of the sea water-based formulation regarding this particular parameter is clearly seen from the results given in Table 4 for the composition of pH= 6 (Example 3) .

In conclusion on stability testings under turbo-accelerated conditions, the composition of the present invention is of significantly higher chemical stability over the control formulation without purified sea water after 28 days test period.

Selected overall data, derived from Tables 3 and 4, are given in Table 7.

**Table 7. Overall stability data of the composition of the present invention over the control formulation without purified sea water, after being stored at turbo-accelerated conditions, i.e. 50 °C/80% RH; after 28 days.**

| **No.** | **Composition** | **Purified sea water** | **Critical parameter** | **Sea water impact on stability** |
|---|---|---|---|---|
| | | | **Assay API 1 (%)** | |
| 1 | Control | No | 96.0 | +2.6% increased API **1** content |
| 2 | of this invention | Yes | 98.5 | |

| | | | **Assay API 2 (%)** | |
|---|---|---|---|---|
| 1 | Control | No | 90.3 | +7.1% increased API **2** content |
| 2 | of this invention | Yes | 96.7 | |

| | | | **Impurity 1A (%)** | |
|---|---|---|---|---|
| 1 | Control | No | 0.31 | 58.1% decreased content of **1A** |
| 2 | of this invention | Yes | 0.13 | |

| | | | **Impurity 2A (%)** | |
|---|---|---|---|---|
| 1 | Control | No | 0.34 | 67.6% decreased content of **2A** |
| 2 | Of this invention | Yes | 0.11 | |

| | | | **Impurity 2C (%)** | |
|---|---|---|---|---|
| 1 | Control | No | 3.39 | 55.8% decreased content of **2C** |
| 2 | of this invention | Yes | 1.50 | |

| | | | **UI¹ (%)** | |
|---|---|---|---|---|
| 1 | Control | No | 0.18 | 27.8% decreased content of **UI** |
| 2 | of this invention | Yes | 0.13 | |

| | | | **TI² (%)** | |
|---|---|---|---|---|
| 1 | Control | No | 3.88 | 54.6% decreased content of **TI** |
| 2 | of this invention | Yes | 1.76 | |

| | | | | |
|---|---|---|---|---|
| ¹ UI= unspecified impurities; ² TI= total impurities. | | | | |

Additional stability testings performed under:
(a) accelerated conditions at 40 °C and 75% RH;
(b) intermediate conditons at 30 °C and 65% RH; and
(c) standard, room storage conditions at 25 °C and 60% RH;
also showed significant improvement of the composition from the present invention (Example 4; pH= 4) in comparison with the control formulation without the purified sea water (Example 2) as can be seen from the Table 8 and 9. Although these were milder test conditions, significant differences can be seen through the critical parameter of individual (UI) and especially total impurities (TI).

**Table 8. Stability data after storage at room temperature conditions (25 °C/60% RH) for 3 months; compositions were adjusted to pH= 4.**

| **Composition** | **Control (Example 2; pH= 4)** | | | **Of this invention (Example 4; pH= 4)** | | |
|---|---|---|---|---|---|---|
| **Time of storage** | **T₀** | **1.5 m¹** | **3 m¹** | **T₀** | **1.5m¹** | **3 m¹** |
| **Assay of API 1** | 98.5 | 100.5 | 98.3 | 100.0 | 100.2 | 99.1 |
| **Assay of API 2** | 97.5 | 97.9 | 98.9 | 99.6 | 98.0 | 99.6 |
| **1A** | 0 | 0 | 0 | 0 | 0 | 0 |
| **1B** | 0 | 0 | 0 | 0 | 0 | 0 |
| **1C** | 0.03 | 0.08 | 0.1 | 0 | 0 | 0 |
| **1D** | 0 | 0 | 0 | 0 | 0 | 0 |
| **2A** | 0.34 | n.a. | n.a. | 0 | 0.02 | 0.04 |
| **OSI²** | 0 | 0 | 0.02 | 0 | 0 | 0 |
| **UI³** | 0.18 | 0.18 | 0.17 | 0 | 0 | 0 |
| **TI⁴** | 0.18 | 0.26 | 0.29 | 0 | 0.02 | 0.04 |

| | | | | | | |
|---|---|---|---|---|---|---|
| ¹ m= months; ² OSI = other single impurity; ³ UI= unspecified impurities; ⁴ TI= total impurities; n.a.= not analysed. | | | | | | |

**Table 9. Stability data after storage at accelerated conditions (40 °C/75% RH) for 3 months; compositions were adjusted to pH= 4.**

| **Composition** | **Control (Example 2; pH= 4)** | | | **Of this invention (Example 4; pH= 4)** | | |
|---|---|---|---|---|---|---|
| **Time of storage** | **T₀** | **1.5 m¹** | **3 m¹** | **T₀** | **1.5 m¹** | **3 m¹** |
| **Assay of API 1** | 98.5 | 98.6 | 97.3 | 100.0 | 100.7 | 99.2 |
| **Assay of API 2** | 97.5 | 96.1 | 97.6 | 99.6 | 98.3 | 99.7 |
| **1A** | 0 | 0 | 0.02 | 0 | 0 | 0 |
| **1B** | 0 | 0 | 0 | 0 | 0 | 0 |
| **1C** | 0.03 | 0.17 | 0.32 | 0 | 0.04 | 0.09 |
| **1D** | 0 | 0 | 0 | 0 | 0 | 0 |
| **2A** | 0.34 | n.a. | 0.60 | 0 | 0 | 0.10 |
| **OSI²** | 0 | 0.06 | 0.10 | 0 | 0.02 | 0.06 |
| **UI³** | 0.18 | 0.17 | 0.17 | 0 | 0 | 0 |
| **TI⁴** | 0.18 | 0.40 | 1.21 | 0 | 0.06 | 0.25 |

| | | | | | | |
|---|---|---|---|---|---|---|
| ¹ m= months; ² OSI = other single impurity; ³ UI= unspecified impurities; ⁴ TI= total impurities; n.a.= not analysed. | | | | | | |

This particular most illustrative parameter of total impurities (TI), that is profoundly expresses even under this relatively mild storage conditions, is given in Table 10.

**Table 10. Stability results of the composition from the present invention (Example 4; pH= 4) in comparison with the control formulation without the purified sea water (Example 2; pH= 4); the content of total impurities (TI).**

| **No.** | **Composition** | **Purified sea water** | **Time & storage conditions** | **TI¹ (%)** | **Sea water impact on stability** |
|---|---|---|---|---|---|
| | | | | | |
| 1 | Control | No | 25 °C/60% RH 3 months | 0.29 | 86% decreased impurities |
| 2 | of this invention | Yes | | 0.04 | |
| | | | | | |
| 1 | Control | No | 40 °C/75% RH 3 months | 1.21 | 80% decreased impurities |
| 2 | of this invention | Yes | | 0.25 | |

| | | | | | |
|---|---|---|---|---|---|
| ¹ TI= total impurities. | | | | | |

It was concluded that purified sea water do significantly stabilize both xylometazoline hydrochloride (**1**) and ipratropium bromide (**2**) in the composition of the present invention, what is essentially not expectable to the person skilled in the art. In this manner, purified sea water does act as a functional excipient, which has a rule not only as a diluent and tonicity adjusting agent, but also as stability-improving excipient, or as chemospecific stabilizer. Tentative mechanism of stabilizing action of certain components of purified sea water is given in the next section.

### Presumable mechanism of stability effect of purified sea water on chemical stability of the composition from the invention

It was shown that purified sea water stabilizes both xylometazoline hydrochloride (**1**) and ipratropium bromide (**2**) against chemical degradation in dilute aqueous solutions such as the composition from the present invention that contains humectant like glycerol and chelating agent such as Na₂EDTA•2H₂O.

Tentative mechanism of stability-improving effect of purified sea water can be explained by increasing stability of ipratropium bromide ester group after forming complex **1b** with magnesium (Mg²⁺) and/or calcium (Ca²⁺) cations, which stabilize the structure against hydrolysis, see Figure 1.

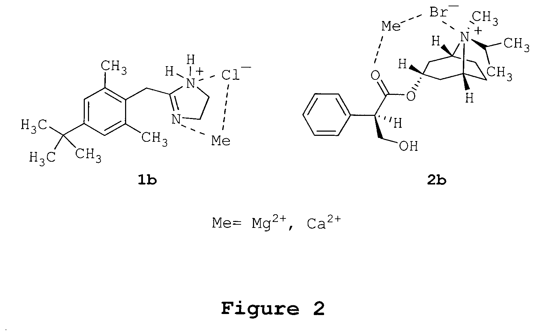

Although observed stabilizing effect of sea salt and its constituents on xylometazoline hydrochloride (1) is less prominent due to generally higher stability of imidazolines against hydrolysis, similar pattern of stabilization, by formation of the complex **2e,** can be valid also in this case, see Figure 2.

Possible structures of stabilizing complexes **1b** and **2e** of ipratropium bromide (2) and xylometazoline hydrochloride (1) with magnesium (Mg²⁺) or calcium (Ca²⁺) cations are shown in Figure 2.

It must be pointed out that such effect is completely unexpected by the person skilled in the arts of both pharmaceutical technology and pharmaceutical chemistry, since it is well known that metal salts, due to certain degree of Lewis acidity, do act as more or less effective catalysts for ester synthesis/hydrolysis. For instance, the following literature references describe catalytic effects of metal salts, e.g. Zn(ClO₄₂•6H₂O and Fe₂(SO₄)₃•xH₂O on synthesis of some ester-group bearing chemical compounds just like is ipratropium bromide (**2**) itself; please see:
(5) G. Bartoli, J. Boeglin, M. Bosco, M. Locatelli, M. Massaccesi, P. Melchiorre, L. Sambri: Highly Efficient Solvent-Free Condensation of Carboxylic Acids with Alcohols Catalysed by Zinc Perchlorate Hexadydrate, Zn(ClO4)2•6H2O, Adv. Synth. Catal. 347 (2005) 33-38; and
(6) G.-S. Zhang: Fe2(SO4))3•xH2O in synthesis: A Convenient and Efficient Catalyst for the Esterification of Aromatic Carboxylic Acids with Alcohols, Synth. Commun. 29 (1999) 607-611.

This metal ion-catalysed ester formation can be equally applied in the ester hydrolysis direction, since the catalysts serves only to faster reach the equilibrium of the reaction:

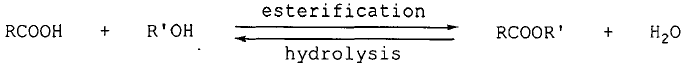

The direction of the reaction, esterification *versus* hydrolysis is only matter of reaction conditions. The reaction conditions within the composition of the present invention include highly diluted aqueous solution, meaning high molar ratio of water molecules against ester group of ipratropium bromide (**2**) or imine moiety of imidazoline group of xylometazoline hydrochloride (**1**). This represents essentially ideal conditions for hydrolysis reaction. Thus, magnesium (Mg²⁺) and calcium (Ca²⁺) ions from the purified sea water, acting as mild Lewis acids, although milder than above-mentioned Zn²⁺ or Fe³⁺, after forming complexes with carbonyl oxygen atoms of ipratropium bromide (**2**) and nitrogen atom of imine moiety of imidazoline group of xylometazoline hydrochloride (**1**), see Figure 1, can catalyse hydrolysis reactions that would led to the formation of degradation products **1A**, **2C,** etc.

Although the catalytic effect of calcium (Ca²⁺) ion on ester hydrolysis is not described in the field of synthetic organic chemistry, it certainly does not mean that the effect does not exist at all. To support this statement, we refer to the scientific paper that teaches about the similar action of some slightly stronger Lewis acids like of magnesium (Mg²⁺) salts or, e.g. Mg(ClO₄)₂ in reaction of carboxylic acids with dialkyl dicarbonates:
(7) L. Grooßen, A. Döhring: Lewis Acids as Highly Efficient Catalysts for the Decarboxylative Esterification of Carboxylic Acids with Dialkyl Dicarbonates, Adv. Synth. Catal. 345 (2003) 943-947.

From this scientific conclusions, the person skilled in the art of pharmaceutical chemistry can easily elucidate that weaker Lewis acids such as magnesium (Mg²⁺) and calcium (Ca²⁺) cations, by activation of carbonyl function of ester group, e.g. in ipratropium bromide (**2**), or imine moiety of imidazoline group of xylometazoline hydrochloride (**1**), would also catalyse such hydrolysis reactions but presumably at lower rate, see Figure 1.

However, in the context of prolonged storage of such formulations, like are all dilute aqueous nasal preparations with declared shelf life of 2-3 years, this hydrolysis catalytic effect of Mg²⁺ and/or Ca²⁺ can be more than significant.

In this manner, person skilled in these arts of pharmaceutical chemistry and pharmaceutical technology would rather expect opposite, de-stabilizing effect of magnesium (Mg²⁺) and calcium (Ca²⁺) cations from purified sea water on chemical stability of ester group of API **2** or imidazoline group of API **1** against hydrolysis.

This conclusion support our statement that our experimental results are completely unexpected by the person skilled in the art.

Furthermore, this support our opinion that the present invention is characterized by significant inventive step that is based on the experimental results of stabilizing effect of purified sea water on chemical stability of xylometazoline hydrochloride (**1**) and ipratropium bromide (**2**) in dilute aqueous solution, wherein it acts as stability-improving excipient.

### The use of the composition from the invention

Due to the known pharmacological actions of xylometazoline hydrochloride (**1**) and ipratropium bromide (**2**), the composition from the present invention is used for manufacturing of medicament for topical treatment of nasal diseases.

Nasal diseases are selected from the group consisting of rhinorrhea, allergic rhinitis, non-allergic (vasomotor) rhinitis, and infective rhinitis. Non-allergic rhinitis, known under the term "runny nose" includes gustatory, autonomic, hormonal, drug-induced, and atrophic rhinitis.

In other words, the nasal diseases that can be treated with the medicament produced from the composition of the present invention are as follows: runny nose, common cold, nasal congestion, and sneezing.

### Examples

### Example 1. Preparation and analysis of purified sea water

Purified sea water was produced by extraction of natural sea water from Adriatic Sea from specific geographic place of the Primorsko-Goranska County (HR). The crude sea water was filtered through the column of clean marine sand in order to remove all mechanical impurities and marine organisms. Furthermore, thus filtered sea water was subjected to a two-step filtration process:
(i) filtration through 10 µm filter; and
(ii) sterile filtration; through 0.2 µm filter.

Thus obtained purified sea water is sterile and suitable for production of pharmaceutical nasal products like those from the present invention. Analysis of purified sea water was performed by ion-chromatography, and the specification of the product regarding the composition of relevant cations and anions are given in Table 1.

### Example 2. Preparation of the control composition of nasal drops without purified sea water

To purified water (80.00 g; 80% w/w), 85% glycerol (2.73 g; 2.73% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to specific pH in region 3-7; e.g. pH= 6 or 4 by addition of with either dilute (0.1 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained nasal drops was in the form of clear, colourless, and odourless aqueous solution of pH= 6 or pH= 4. These samples are used as control compositions for stability testings described in Example 12.

### Example 3. Preparation of the composition from the present invention in the form of nasal drops

Purified sea water was filtered through 0.2 µm resin-bonded glass fiber filter. To purified water (80.00 g; 80% w/w), filtered, purified sea water (10.00 g; 10% w/w) and 85% glycerol (2.73 g; 2.73% w/w) were added and homogenized by stirring for 5 minutes. Thus obtained solution was filtered through the 1.2 µm polypropylene (PP) filter. Then, disodium edetate dihydrate (0.05 g; 0.05% w/w) was added and dissolved by stirring for 5 minutes. Afterwards, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 6 by addition of with either dilute (0.1 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained composition of nasal drops was in the form of clear, colourless, and odourless aqueous solution of pH= 6, and measured osmolality was 385 (±3%) mOsm/kg.

### Example 4. Preparation of the composition from the present invention in the form of nasal drops with specific pH values from the range pH 3-7.

Purified sea water was filtered through 0.2 µm resin-bonded glass fiber filter. To purified water (80.00 g; 80% w/w), filtered, purified sea water (10.00 g; 10% w/w) and 85% glycerol (2.73 g; 2.73% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 3, 4, 5, and 7 by addition of with either dilute (0.1 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained composition of nasal drops was in the form of clear, colourless, and odourless aqueous solutions. These samples are used as compositions from this invention for stability testings at various pH values 3, 4, 5, and 7 as described in Example 12.

### Example 5. Preparation of the composition from the present invention in the form of nasal drops

Purified sea water was filtered through 0.2 µm resin-bonded glass fiber filter. To purified water (60.00 g; 60% w/w), filtered, purified sea water (25.00 g; 25% w/w) and 85% glycerol (5.88 g; 5.88% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 3.2 by addition of with either dilute (0.5 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained composition of nasal drops was in the form of clear, colourless, and odourless aqueous solution of pH= 3.2, and measured osmolality was 820 (±3%) mOsm/kg.

### Example 6. Preparation of the composition from the present invention in the form of nasal drops

Purified sea water was filtered through 0.2 µm resin-bonded glass fiber filter. To purified water (80.00 g; 80% w/w), filtered, purified sea water (5.00 g; 5% w/w) and 85% glycerol (2.73 g; 2.73% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 4 by addition of with either dilute (0.5 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained composition of nasal drops was in the form of clear, colourless, and odourless aqueous solution of pH= 4, and measured osmolality was 270 (±3%) mOsm/kg.

### Example 7. Preparation of the composition from the present invention in the form of liquid nasal spray

Purified sea water was filtered through 0.2 µm resin-bonded glass fiber filter. To purified water (80.00 g; 80% w/w), filtered, purified sea water (5.00 g; 5% w/w) and 85% glycerol (2.73 g; 2.73% w/w) were added and homogenized by stirring for 5 minutes. Then, disodium edetate dihydrate (0.05 g; 0.05% w/w) was added and dissolved by stirring for 5 minutes. Then, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter. Afterwards, the pH value of thus prepared solution was determined, and subsequently corrected to pH= 6.5 by addition of with either dilute (0.5 mol/dm³) solution of hydrochloric acid (HCl) or sodium hydroxide (NaOH).

The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained nasal spray was in the form of clear, colourless, and odourless aqueous solution of pH= 6.5. The solution was filled into 10 ml plastic (PE-HD) bottles with spraying device or alternatively filled system for nasal aerosols.

### Example 8. Preparation of the composition from the present invention in the form of liquid nasal spray

Purified sea water was filtered through 0.2 µm resin-bonded glass fiber filter. To purified water (80.00 g; 80% w/w), filtered, purified sea water (10.00 g; 10% w/w), 85% glycerol (2.35 g; 2.35% w/w), and d-panthenol (0.50 g; 0.5% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.10 g; 0.1% w/w) was added and dissolved by stirring for 5 minutes. Then, ipratropium bromide monohydrate (**2**; 0.105 g; 0.105% w/w; corresponds to 0.1% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 3 by addition of with either dilute (0.5 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained nasal spray was in the form of clear, colourless, and odourless aqueous solution of pH= 3. The solution was filled into 10 ml plastic (PE-HD) bottles with spraying device or alternatively filled into system for nasal aerosols.

### Example 9. Preparation of the composition from the present invention in the form of nasal wash

Purified sea water was filtered through 0.2 µm resin-bonded glass fiber filter. To purified water (80.00 g; 80% w/w), filtered, purified sea water (15.00 g; 15% w/w), 85% glycerol (2.35 g; 2.35% w/w), and d-panthenol (0.50 g; 0.5% w/w) were added and homogenized by stirring for 5 minutes. Afterwards, disodium edetate dihydrate (0.05 g; 0.05% w/w) was added and dissolved by stirring for 5 minutes. Then, xylometazoline hydrochloride (**1**; 0.05 g; 0.05% w/w) was added and dissolved by stirring for 5 minutes. Then, ipratropium bromide monohydrate (**2**; 0.063 g; 0.063% w/w; corresponds to 0.06% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 4.2 by addition of with either dilute (0.5 mol/dm³) solution of sulfuric acid (H₂SO₄)or potassium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus prepared nasal wash was in the form of clear, colourless, and odourless aqueous solution of pH= 4.2. The solution was filled into 100 ml plastic (PE-HD) bottles.

### Example 10. Preparation of the composition from the present invention in the form of nasal wash

Purified sea water was filtered through 0.2 µm resin-bonded glass fiber filter. To purified water (80.00 g; 80% w/w), filtered, purified sea water (10.00 g; 10% w/w), 85% glycerol (2.73 g; 2.73% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.01 g; 0.01% w/w) was added and dissolved by stirring for 5 minutes. Then, ipratropium bromide monohydrate (**2**; 0.0105 g; 0.0105% w/w; corresponds to 0.01% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 5.8 by addition of with either dilute (0.5 mol/dm³) solution of hydrochloric acid (HCl) or sodium hydroxide (NaOH). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus obtained nasal wash was in the form of clear, colourless, and odourless aqueous solution of pH= 5.8. The solution was filled into 100 ml plastic (PE-HD) bottles.

### Example 11. Preparation of the composition from the present invention in the form of nasal wash

Purified sea water was filtered through 0.2 µm resin-bonded glass fiber filter. To purified water (80.00 g; 80% w/w), filtered, purified sea water (5.00 g; 5% w/w), 85% glycerol (2.35 g; 2.35% w/w), d-panthenol (0.50 g; 0.5% w/w), and ectoin (0.50 g; 0.5% w/w) were added and homogenized by stirring for 5 minutes. Then, disodium edetate dihydrate (0.05 g; 0.05% w/w) was added and dissolved by stirring for 5 minutes. Afterwards, xylometazoline hydrochloride (**1**; 0.01 g; 0.01% w/w) was added and dissolved by stirring for 5 minutes. Then, ipratropium bromide monohydrate (**2**; 0.0105 g; 0.0105% w/w; corresponds to 0.01% w/w of anhydrous ipratropium bromide) was added and dissolved by stirring for 5 minutes. Thus obtained solution was filtered through the 1.2 µm polypropylene (PP) filter.

Then, the pH value of thus prepared solution was determined, and subsequently corrected to pH= 7 by addition of with either dilute (0.5 mol/dm³) solution of sulphuric acid (H₂SO₄)or sodium carbonate (Na₂CO₃). The resulting solution was further diluted with purified water up to the total weight of 100.00 g (100% w/w). Final solution was subjected to filtration through 0.2 µm polyvinylidene difluoride (PVDF) filter to reduce bioburden, followed by sterile filtration through 0.1 µm PVDF filter.

Thus prepared nasal wash was in the form of clear, colourless, and odourless aqueous solution of pH= 7. The solution was filled into 100 ml plastic (PE-HD) bottles.

### Example 12: Stability study of the composition from the present invention

The stability of the composition of this invention was studied by monitoring of degradation products that appeared under:
(a) turbo-accelerated conditions at 50 °C and 80% of relative humidity (RH);
(b) accelerated studies at 40°C and 75% RH;
(c) intermediate studies at 30 °C and 65% RH; and
(d) standard, room conditions studies at 25 °C and 65% RH;
as common in the pharmaceutical industry.

Stability testings were performed on:
(i) control composition without purified sea water of pH= 6 and 4; product from Example 2;
(ii) the composition with purified sea water of pH= 6; product from Example 3; and
(iii) the compositions with purified sea water of pH values of 3, 4, 5, and 7; products from Example 4.

Chemical compositions of the control formulation and model formulations of this invention, from Examples 2 and 3 were exactly the same, except absence (Example 2) or presence (Example 3) of purified sea water, see Table 2.

The samples are tested against several critical stability parameters:
(1) pH value of the tested sample;
(2) assay of xylometazoline hydrochloride (**1**);
(3) assay of ipratropium bromide (**2**);
as well as quantitative determination of impurities (related substances) generated during the course of degradation process:
(4) *N*-(2-aminoethyl-2-[4-(1,1-dimethylethyl)-2,6-dimethylphenyl] acetamide (**1A**);
(5) (1*R*,3*r*,5*S*,8*r*)-3-hydroxy-8-methyl-8-(1-methylethyl)-8-azonia bicyclo[3.2.1]octane (**2A**);
(6) (1*R*,3*r*,5*S*,8*s*)-3-[[(2*RS*)-3-hydroxy-2-phenylpropanoyl]oxy]-8-methyl-8-(1-methylethyl)-8-azoniabicyclo[3.2.1]octane (**2B**);
(7) (2*RS*)-3-hydroxy-2-phenylpropanoic acid (**2C**), known under generic name DL-tropic acid;
(8) 2-phenylpropenoic acid (**2D**), known under generic name atropic acid;
(9) unspecified impurities (UI); as well as
(10) total impurities (TI).

The analytical method for assay of xylometazoline hydrochloride (**1**) and ipratropium bromide (**2**) in the final composition is based on quantitative ultra high performance liquid chromatography (UHPLC). It uses the HSS C18 column; phosphate buffer pH= 3.0 : acetonitrile = 90:10 V/V, as mobile phase A, and 100% acetonitrile as mobile phase B; gradient program is set to 100% A at start, following 15% B, and then again 100% A in the end. Detection is carried out by means of photodiode array (PDA) detector at 206 nm; retention times (*t*_{R}) of APIs were: *t*_{R} (**1**) ≈ 6.0 min, *t*_{R} (**2**) ≈ 2.0 min.

The analytical method for assay of xylometazoline hydrochloride (**1**) and ipratropium bromide (**2**) impurities **1A, 2B, 2C, 2D,** other known and unknown impurities, and total impurities of **1** and **2** in the final composition is based on quantitative ultra high performance liquid chromatography (UHPLC). It uses the HSS C18 column; phosphate buffer pH= 3.0 : acetonitrile = 90:10, V/V, as mobile phase A and solvent, and phosphate buffer pH= 3.0 : acetonitrile = 70:30 V/V, as a mobile phase B; gradient program is set to 100% A at start, following 100% B, and then again 100% A in the end. Detection is carried out by means of PDA detector at 206 nm; retention times (*t*_{R}) were as follows: *t*_{R} (**1**) ≈ 13.7 min, *t*_{R} (**1A**) ≈ 14.4 min, *t*_{R} (**2**) ≈ 3.7 min, *t*_{R} (**2A**) ≈ 4.1 min, *t*_{R} (**2B**) ≈ 5.1 min, *t*_{R} (**2C**) ≈ 2.5 min, *t*_{R} (**2D**) ≈ 10.3 min.

The analytical method for assay of impurity **2A** in the final composition is based on quantitative ultra high performance liquid chromatography (UHPLC). It employs hydrophilic interaction chromatography (HILIC)-based column; water : acetonitrile = 5:95, v/v + 0.315 g/l of ammonium formate as mobile phase A, and 0.1% aqueous solution formic acid as mobile phase B; gradient program is set to 100% A at start, following 30% B, and then again 100% A in the end. Detection is carried out by means of MS detector at 184 Da, positive polarity. Due to the MS detection, the method is highly specific and can be used for unambiguous identification of impurity **1A.**

Results of stability study of the composition from the present invention (Example 3) against the control formulation without the purified sea water (Example 2), both of pH= 6, performed under turbo-accelerated conditions at 50 °C and 80% RH, are presented in Tables 3 and 4.

Stability data of the composition according to the present invention (Example 4) over various pH values of 3, 4, 5, and 7, that are obtained from the study performed under turbo-accelerated conditions at 50 °C and 80% RH, are given in Tables 5 and 6.

Overall stability data of the composition of the present invention (Example 3) over the control formulation without purified sea water (Example 2) obtained under turbo-accelerated conditions (50 °C/80% RH), are presented in Table 7.

Stability data of the composition of the present invention (Example 4) over the control formulation without purified sea water (Example 2), both of pH= 4, obtained under standard, room temperature conditions (25 °C/60% RH) during 3 months storage are shown in Table 8.

Stability data of the composition of the present invention (Example 4) over the control formulation without purified sea water (Example 2), both of pH= 4, obtained under accelerated conditions (40 °C/75% RH) during 3 months storage are presented in Table 9.

Overall stability data of the composition of the present invention (Example 4) over the control formulation without purified sea water (Example 2), both of pH= 4, obtained under standard, room temperature (25 °C/60% RH) and accelerated conditions (40 °C/75% RH) are given in Table 10.

### Industrial Applicability

This invention is used for manufacturing of pharmaceutical formulation for nasal use of increased stability based on sea water as functional, stability-improving ingredient. Therefore, industrial applicability of this invention is obvious.

## Claims

1. A nasal composition of improved stability, consisting of:
(i) xylometazoline hydrochloride (**1**), 0.01-0.1% w/w;
(ii) ipratropium bromide (**2**), 0.01-0.1% w/w; 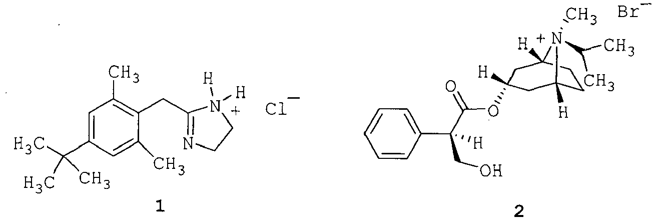 and
(iii) pharmaceutical excipients required to form final dosage forms selected from the group consisting of: nasal drops, liquid nasal sprays, nasal aerosols or nasal washes;
**characterized by that** said nasal composition comprises the content of purified sea water as functional, stability-improving excipient, from 5-25% w/w; and where said nasal composition has the pH value within the range 3-7, and said nasal composition has osmolality within the range 270-820 mOsm/kg.

2. A nasal composition of improved stability according to claim 1, **characterized by that** the pH value is within the range 3-4.2 or 5.8-7.0.

3. A nasal composition of improved stability according to claim 1, **characterized by** the following ingredients and parameter selection:
(i) xylometazoline hydrochloride (**1**), 0.05% w/w;
(ii) ipratropium bromide (**2**), 0.06% w/w;
(iii) purified sea water as functional, stability-improving excipient, 10% w/w;
(iv) pharmaceutical excipients required to form final dosage form of nasal drops or liquid nasal spray;
(v) with the pH value within the range 3.2-4.2.

4. A process for production of nasal composition according to any of the claims 1-3, **characterized by** the following steps:
(i) filtration of purified sea water through 0.2 µm filter;
(ii) mixing of filtered, purified sea water with predominant part of purified water;
(iii) dissolution of excipients: humectants and chelating agents;
(iv) dissolution of active pharmaceuticals ingredients (APIs): xylometazoline hydrochloride (**1**) and ipratropium bromide (**2**) ;
(v) filtration through 1.2 µm filter;
(vi) determination of pH value of thus prepared solution;
(vii) correction of pH to defined value by addition of dilute solution of pharmaceutically acceptable acids or pharmaceutically acceptable bases;
(viii) addition of remaining part of purified water up to the final mass of the batch;
(ix) filtration through 0.2 µm filter for bioburden reduction;
(x) sterile filtration through 0.1 µm filter; and
(xi) filling the filtered liquid composition into containers.

5. A process for production of nasal composition according to claim 4, wherein the selected excipients, humectants and chelating agents, are glycerol and disodium edetate.

6. A process for production of nasal composition according to claim 4 or 5, wherein the selected pharmaceutically acceptable acids or pharmaceutically acceptable bases are HCl or NaOH.

7. Formulation according to any of the claims 1-3, for use as a medicament for topical treatment of nasal diseases.

8. Formulation according to the claim 7, where nasal diseases are selected from the group consisting of: rhinorrhea, allergic rhinitis, non-allergic rhinitis, and infective rhinitis.

## Patentansprüche

1. Nasale Zusammensetzung mit verbesserter Stabilität, bestehend aus:
(i) Xylometazolinhydrochlorid (**1**)**,** 0,01-0,1 % Gew./Gew.;
ii) Ipratropiumbromid (**2**), 0,01-0,1 % Gew./Gew.; 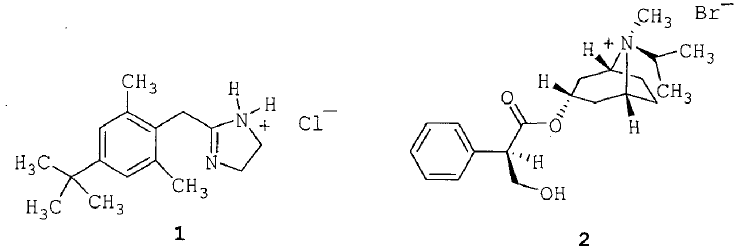 und
(iii) pharmazeutischen Hilfsstoffen, die zur Herstellung von Fertigdosierungsformen erforderlich sind, ausgewählt aus der Gruppe, bestehend aus: Nasentropfen, flüssigen Nasensprays, Nasenaerosolen oder Nasenspülungen;
**dadurch gekennzeichnet, dass** die nasale Zusammensetzung den Gehalt an gereinigtem Meerwasser als funktionellem, stabilitätsverbesserndem Hilfsstoff von 5-25 % Gew./Gew.umfasst; und wobei die nasale Zusammensetzung den pH-Wert im Bereich von 3-7 aufweist und die nasale Zusammensetzung eine Osmolalität im Bereich von 270-820 mOsm/kg aufweist.

2. Nasale Zusammensetzung mit verbesserter Stabilität nach Anspruch 1, **dadurch gekennzeichnet, dass** der pH-Wert im Bereich von 3-4,2 oder 5,8-7,0 liegt.

3. Nasale Zusammensetzung mit verbesserter Stabilität nach Anspruch 1, **gekennzeichnet durch** die folgenden Inhaltsstoffe und Parameterauswahl:
(i) Xylometazolinhydrochlorid (**1**), 0,05 % Gew./Gew.;
(ii) Ipratropiumbromid (**2**), 0,06 % Gew./Gew.;
(iii) gereinigtes Meerwasser als funktioneller, stabilitätsverbessernder Hilfsstoff, 10 % Gew./Gew.;
(iv) pharmazeutische Hilfsstoffe, die zur Herstellung der Fertigdosierungsform von Nasentropfen oder flüssigem Nasenspray erforderlich sind;
(v) mit dem pH-Wert im Bereich von 3,2-4,2.

4. Verfahren zur Herstellung einer nasalen Zusammensetzung nach einem der Ansprüche 1-3, **gekennzeichnet durch** die folgenden Schritte:
(i) Filtration von gereinigtem Meerwasser durch 0,2 µm-Filter;
(ii) Mischen von filtriertem, gereinigtem Meerwasser mit dem überwiegenden Teil des gereinigten Wassers;
(iii) Auflösung von Hilfsstoffen: Feuchthaltemitteln und Chelatbildnern;
(iv) Auflösung von pharmazeutischen Wirkstoffen (APIs): Xylometazolinhydrochlorid (**1**) und Ipratropiumbromid (**2**);
(v) Filtration durch 1,2 µm-Filter;
(vi) Bestimmung des pH-Wertes der so hergestellten Lösung;
(vii) Korrektur des pH-Wertes auf einen definierten Wert durch Zugabe einer verdünnten Lösung von pharmazeutisch verträglichen Säuren oder pharmazeutisch verträglichen Basen;
(viii) Zugabe des verbleibenden Teils des gereinigten Wassers bis zum Fertiggewicht der Charge;
(ix) Filtration durch 0,2 µm-Filter zur Verminderung der Biobelastung;
(x) Sterile Filtration durch 0,1 µm-Filter; und
(xi) Abfüllen der filtrierten flüssigen Zusammensetzung in Behälter.

5. Verfahren zur Herstellung einer nasalen Zusammensetzung nach Anspruch 4, wobei die ausgewählten Hilfsstoffe, Feuchthaltemittel und Chelatbildner, Glycerin und Dinatriumedetat sind.

6. Verfahren zur Herstellung einer nasalen Zusammensetzung nach Anspruch 4 oder 5, wobei die ausgewählten pharmazeutisch verträglichen Säuren oder pharmazeutisch verträglichen Basen HCl oder NaOH sind.

7. Formulierung nach einem der Ansprüche 1-3 zur Verwendung als Medikament zur topischen Behandlung von Nasenerkrankungen.

8. Formulierung nach Anspruch 7, wobei Nasenerkrankungen ausgewählt sind aus der Gruppe, bestehend aus: Rhinorrhö, allergischer Rhinitis, nicht-allergischer Rhinitis und infektiöser Rhinitis.

## Revendications

1. Composition nasale ayant une stabilité améliorée, constituée par :
(i) du chlorhydrate de xylométazoline (1), 0,01 à 0,1 % p/p ;
(ii) du bromure d'ipratropium (2), 0,01 à 0,1 % p/p ; 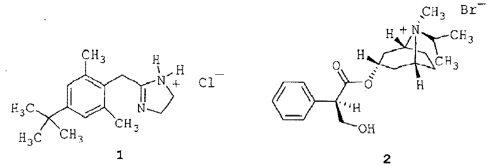 et
(iii) les excipients pharmaceutiques requis pour former les formes galéniques finales sélectionnées à partir du groupe constitué par : des gouttes nasales, des pulvérisations nasales liquides, des aérosols nasaux ou des lavages nasaux ;
**caractérisée en ce que** ladite composition nasale comprend le contenu d'eau de mer purifiée en tant qu'excipient fonctionnel d'amélioration de stabilité, de 5 à 25 % p/p ; et où la valeur du pH de ladite composition nasale se trouve dans la plage de 3 à 7, et ladite composition nasale a une osmolalité dans la plage de 270 à 820 mOsm/kg.

2. Composition nasale ayant une stabilité améliorée selon la revendication 1, **caractérisée en ce que** la valeur du pH se trouve dans la plage de 3 à 4,2 ou de 5,8 à 7,0.

3. Composition nasale ayant une stabilité améliorée selon la revendication 1, **caractérisée par** les ingrédients et le choix de paramètres suivants :
(i) chlorhydrate de xylométazoline (1), 0,05 % p/p ;
(ii) bromure d'ipratropium (2), 0,06 % p/p ;
(iii) eau de mer purifiée en tant qu'excipient fonctionnel d'amélioration de stabilité, 10% p/p ;
(iv) excipients pharmaceutiques requis pour former la forme galénique finale de gouttelettes nasales ou de pulvérisation nasale liquide ;
(v) avec la valeur du pH dans la plage de 3,2 à 4,2.

4. Procédé de production d'une composition nasale selon l'une quelconque des revendications 1 à 3, **caractérisé par** les étapes suivantes :
(i) filtration d'eau de mer purifiée à travers un filtre de 0,2 µm ;
(ii) mélange d'eau de mer purifiée et filtrée avec une partie prédominante d'eau purifiée ;
(iii) dissolution d'excipients : humectants et agents chélateurs ;
(iv) dissolution d'ingrédients pharmaceutiques actifs (API) : chlorhydrate de xylométazoline (1) et bromure d'ipratropium (2) ;
(v) filtration à travers un filtre de 1,2 µm ;
(vi) détermination de la valeur du pH de la solution ainsi préparée ;
(vii) correction de la valeur du pH à une valeur définie par ajout d'une solution diluée d'acides pharmaceutiquement acceptables ou de bases pharmaceutiquement acceptables ;
(viii) ajout de la partie restante d'eau purifiée jusqu'à la masse finale du lot ;
(ix) filtration à travers un filtre de 0,2 µm pour réduire la charge microbienne ;
(x) filtration stérilisante à travers un filtre de 0,1 µm ; et
(xi) remplissage de la composition liquide filtrée dans des récipients.

5. Procédé de production d'une composition nasale selon la revendication 4, dans lequel les excipients, humectants et agents chélateurs sélectionnés, sont le glycérol et l'édétate disodique.

6. Procédé de production d'une composition nasale selon la revendication 4 ou 5, dans lequel les acides pharmaceutiquement acceptables ou les bases pharmaceutiquement acceptables sont le HCl et le NaOH.

7. Formulation selon l'une quelconque des revendications 1 à 3, pour son utilisation en tant que médicament pour le traitement topique de maladies nasales.

8. Formulation selon la revendication 7, dans laquelle les maladies sont sélectionnées à partir du groupe constitué par : la rhinorrhée, la rhinite allergique, la rhinite non allergique, et la rhinite infectieuse.
